# EUROPEAN PATENT SPECIFICATION

(11) **EP 0 630 412 B1**
(45) Date of publication and mention of the grant of the patent: **11.11.1998**
(21) Application number: 92911140.9
(22) Date of filing: 29.04.1992
(51) Int. Cl.: C12Q 1/48, A01N 29/00, A01N 29/10, A61K 31/095, A61K 31/135, G01N 33/574, G01N 33/573, G01N 33/50

(54) **METHODS TO PROFILE AND TREAT ABNORMAL CELLS**
VERFAHREN ZUR ERKENNUNG UND BEHANDLUNG ANOMALER ZELLEN
PROCEDE D'IDENTIFICATION ET DE TRAITEMENT DE CELLULES ANORMALES

(30) Priority: 29.04.1991 US 693245
(43) Date of publication of application: 28.12.1994
(73) Proprietor: TERRAPIN TECHNOLOGIES, INC., San Francisco, CA 94103 (US)
(72) Inventor: KAUVAR, Lawrence, M., San Francisco, CA 94117 (US)
(74) Representative: Goldin, Douglas Michael
(86) International application number: US9203537
(87) International publication number: WO9219767

(56) References cited:
- WO-A-86/00991
- PATENT ABSTRACTS OF JAPAN vol. 10, no. 56 (C-331)(2113) 6 March 1986 & JP-A-60 199 384 (KIYOMI SATOU) 8 October 1985
- CANCER CHEMOTHERAPY AND PHARMACOLOGY vol. 33, no. 1 , 1993 , WASHINGTON DC USA pages 63 - 70 J.E. FLATGAARD ET AL. 'Isozyme specificity of novel glutathione-s-transferase'
- Cancer Research, Volume 50, issued 15 October 1990, D.J. WAXMAN, "Glutathione S-Transferases: Role in Alkylating Agent Resistance and Possible Target for Modulation Chemotherapy - A Review", pages 6449-6454, see entire document.
- Proc. Natl. Acad. Sci. USA, Volume 82, issued November 1985, B. MANNERVIK et al., "Identification of Three Classes of Cytosolic Glutathione Transferase Common to Several Mammalian Species: Correlation Between Structural Data and Enzymatic Properties", pages 7202-7206, see entire document.
- Cancer Research, Volume 48, issued 01 July 1988, K.D. TEW et al., "Ethacrynic Acid and Piriprost as Enhancers of Cytotoxicity in Drug Resistant and Sensitive Cell Lines", pages 3622-3625, see entire document.
- Carcinogenesis, Volume 11, issued September 1990, V.M. CASTRO et al., "Difference Among Human Tumor Cell Lines in the Expression of Glutathione Transferases and other Glutathione-linked Enzymes", pages 1569-1576, see entire document.

## Description

### Technical Field

The invention relates to a method to manipulate the metabolism of tumor cells differentially from the metabolism of normal cells. More particularly, it concerns altering the activity of tumor-specific glutathione S-transferase (GST) enzymes.

### Background Art

Glutathione S-transferases (GSTs) are a group of isoenzymes which regulate the metabolism and detoxification of foreign substances introduced into cells. In general, GST can facilitate detoxification of foreign substances by a number of mechanisms and can also convert certain precursors into toxic substances.

For example, Waxman, D.J., Cancer Res (1990) 50:6449-6454, has reviewed these functions. As summarized in this report, GST can detoxify lipophilic substances which contain an electrophile by catalyzing the coupling of the electrophilic portion to glutathione (GSH), thus rendering a significant portion of the molecule more polar and susceptible to clearance. A number of the drugs used in chemotherapy are susceptible to this type of detoxification, including, for example, various mustards. A second mechanism resides in the reduction of peroxides with the concomitant oxidation of glutathione; a third simply involves the association of a ligand with the GST in a noncovalent association. Conversely, GST also catalyzes some reactions which convert nontoxic precursors to toxic agents. For example, GST is capable of converting 1,2-dibromoethane and azathioprine to toxins.

Because of the central role of GST in regulating the toxic effects of foreign substances, it is evident that manipulating the GST activity in cells which are subjected to toxic agents intended to kill them will influence the effectiveness of this treatment. This has been demonstrated in a number of studies. For example, Tew, K.D., et al., Cancer Res (1988) 48:3622-3635, showed that ethacrynic acid and piriprost potentiate the cytotoxic activity of chlorambucil in rat and human tumor cell lines. Ethacrynic acid and piriprost are known to affect GST (ethacrynic acid is a diuretic plant phenol; piriprost is a prostaglandin analog). Chlorambucil is a known alkylating agent which is thought to be cleared from cells by the mediation of GST activity to derivatize chlorambucil to GSH; it has, in fact, been demonstrated that chlorambucil is a substrate for GST. The ability of ethacrynic acid and piriprost to inhibit GST activity was thus considered responsible for their ability to enhance the potency of chlorambucil in both chlorambucil-sensitive and chlorambucil-resistant cell lines, although considerable variation in the effectiveness of these protocols was found among the cell lines tested.

At present, there is a multicenter study planned to evaluate the chemosensitizing effect of ethacrynic acid in combination with administration of chlorambucil in patients with chronic lymphocytic leukemia (CLL) that were resistant to therapy using chlorambucil and prednisolone. The selection of patients for this study is based on their demonstrated resistance to treatment with chlorambucil and prednisolone absent the coadministration of ethacrynic acid.

The activity of GST also affects the radiosensitivity of mammalian cells. A problem encountered in treating tumors using radiation resides in the hypoxic condition of many tumor cells as compared to normal cells. Because of their reduced state, these cells are evidently more capable of dealing with the peroxide and other free radicals generated by radiation, and are thus, perversely, more resistant to radiation treatment than their normal counterparts. Use of radiosensitizing oxidizing agents in conjunction with radiation therapy is already well established. A similar result, however, can be obtained by use of agents which deplete intracellular GSH through its catalytic oxidation mediated by GST. Held, K.D., et al., Radiation Res (1988) 115:495-502, showed that dimethyl fumarate, which depletes GSH by covalent bond formation in a reaction mediated by GST, is an effective radiosensitizer in hypoxic cells, the enhancement ratio being much greater than shown in aerobic cells.

Of course, attempts to expose certain cells to toxic protocols preferentially to other cells by manipulating the GSH pathway would be of limited utility if the response of all cells to such manipulation were identical. This situation is illustrated by the use of the drug BSO, for example, that blocks de novo synthesis of GSH and is thus theoretically an effective radiosensitizer. This drug has some preferential effect on tumor cells due to small differences in bioavailability and pharmacokinetics. Similar considerations apply to efforts to manipulate glutathione reductase, the major enzyme involved in recycling oxidized glutathione.

Conferring susceptibility on a certain class of cells, for example tumor cells, in a clearly preferential manner depends on there being some real difference in the response of the glutathione pathway in tumor cells as compared to that in normal cells. Fortunately, one major component of the glutathione pathway, "GST," represents not a single, invariant enzyme but a class of isoenzymes which differ in specific binding abilities, in substrate and inhibitor specificities and in tissue distribution.

The various GST isozymes are dimeric proteins formed by binary combinations of monomers encoded by at least 15 known genes in 4 gene families, resulting in the theoretical possibility of several dozen different dimers even allowing for the preferential dimerization of monomers from the same gene family. In addition to the variability that arises from these combinatorial possibilities, the GST isoenzyme subunits are polymorphic in the human population and have been considered to be subject to additional variation due to gene conversion events among the tandemly repeated members of the family. Posttranslational modifications add further to this variability. Thus, the GST's obtained from tissue samples of a given individual may not necessarily exactly match those of any previously known GST. Particular cell types typically express only a few of these many forms. The particular GST complement in a cell is probably also affected by exposure to certain environmental factors, such as chemical substances, since the enzymes are inducible. Thus, GST represents a family of isoenzymes which differ in quantity and enzymatic and physical properties in various cell types and in various individuals.

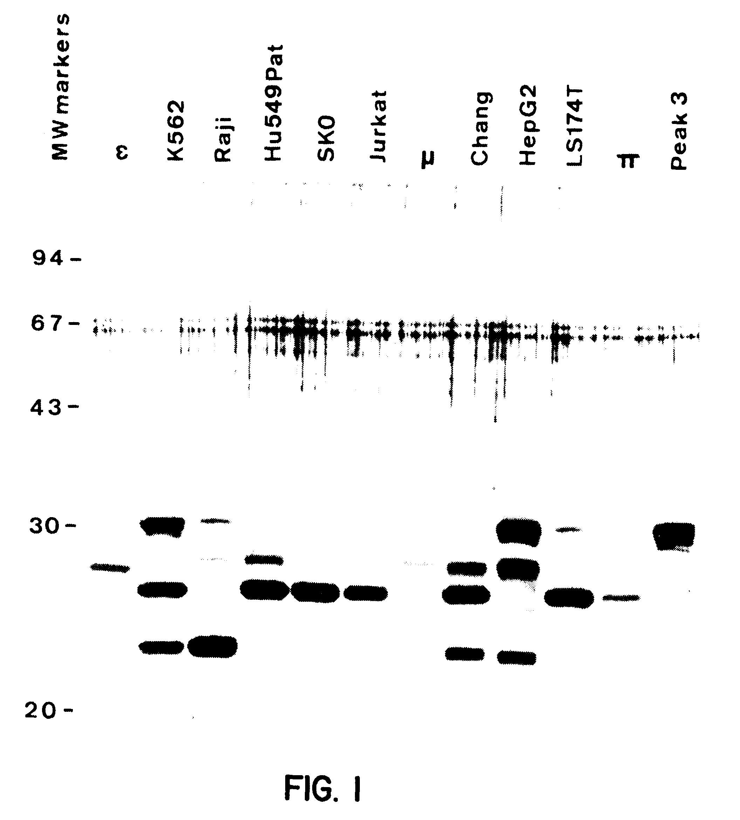

The situation with respect to complement of GST found in various cells is illustrated in Figure 1, which is reproduced from Castro et al., Carcinogenesis (1990) 11:1569 1596. This figure shows the results of SDS gel electrophoretic separations, using silver staining for detection, of soluble GSTs that had been purified on an S-hexylglutathione Sepharose™ column. The various lanes in the gel show the complements of isoenzymes in various tumor cell lines, as well as purified representatives of the major gene families. As shown in Figure 1, the distinctive GST complement of each tumor cell line represents a potential therapeutic target. However, it is difficult to extrapolate from a determination of GST complement determined in this manner, which deactivates the enzyme, to a reactivity profile for the cell line, since genetic polymorphisms and posttranslational modifications may alter reactivity without altering electrophoretic mobility, or, conversely, alter physical properties without affecting reactivity.

Mannervik, B., et al., Proc Natl Acad Sci (1985) 82:7202-7206, propose a classification scheme for GST dividing the groups into a single microsomal subclass and three cytosolic subclasses. These classes show differences in structure, immunological activity, substrate specificity and inhibitor sensitivities. Mannervik, B., et al., describe some of the qualitative properties in detail. For example, the turnover rates in µM/min/mg for ethacrynic acid substrate show a range of 0.01-1.2 for α enzymes; 0.08-0.6 for µ enzymes; and 0.9-4 for π enzymes. On the other hand, δ-5-androstene-3,17-dione shows a high turnover rate for the α subclass, 0.04-8, and much reduced turnover rates for the remaining two subclasses. In the substrates surveyed in this report, higher turnover rates were observed for the α subclass for cumene hyperoxide and δ-5-androstene-3,17-dione; for the µ subclass by bromosulfophthalein, and trans-4-phenyl-3-butene-2-one; and for the π subclass by ethacrynic acid. Similar differences were found with respect to inhibitors. Some members of the α subclass were extremely sensitive to hematin; there was less sensitivity by the members of the µ and π subclasses. The µ subclass was highly sensitive in comparison to the others to Cibacron blue and triphenyltin chloride. Differences in cross-reactivity with regard to antibodies prepared to various members of the subclasses was also demonstrated.

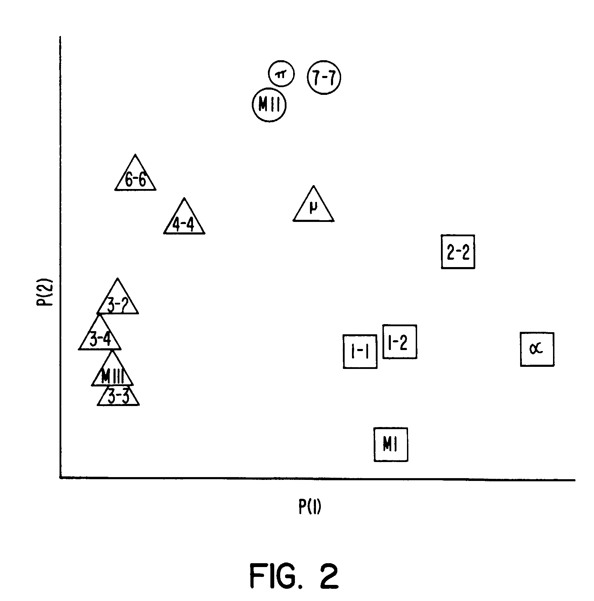

Mannervik further classified the GST isoenzymes that were studied by multiparametric statistics as illustrated in Figure 2, which is reproduced from this article. The purified homo- and heterodimeric GSTs were prepared from several tissue sources in various animal species and profiles of reactivity which described the substrate specificity and inhibitor sensitivity as set forth in the previous paragraph were determined against a panel of 20 small organic molecules. Based on similar functional properties, the purified isoenzymes were grouped into the three families set forth above, which have subsequently been correlated with individual gene families. Figure 2 shows the result of factor analysis which was used to generate the principal components of the distribution (P1) and (P2) and the figure shows the graphical result of this analysis.

The ability of different purified rat GST enzymes to detoxify the 1,3-bis-(2-chloroethyl)-1-nitrosourea (BCNU) by denitrosation was studied by Smith, M.T., et al., Cancer Res (1989) 49:2621-2625. The reported results indicated that µ isoenzymes containing subunit 4 are the best catalysts for this reaction while several members of the α subclass had very weak activity.

An additional aspect of variability among the members of the group of GST isoenzymes resides in differences in their levels of response to various inducers. In an article by Vos, R.M.E., et al., Biochem Pharmacol (1988) 37:1077-1082, the patterns of response in terms of levels of production of the various GST isoenzymes was shown to four different inducers-hexachlorobenzene, benzyl isothiocyanate, phenobarbital, and 3-methylcholanthrene. In this study, rats were treated with the candidate inducers and the GST isoenzymes of the liver were purified on S-hexylglutathione Sepharose™ 6B and then separated using FPLC-chromatofocusing. Both hexachlorobenzene and phenobarbital caused an increase in the relative concentration of isoenzymes designated 1-1 and 3-3 by Vos, as well as a decrease in the relative amounts of isoenzymes 2-2 and 4-4. These inducers increased the relative amounts of protein represented by subunits 1 and 3 when compared to subunits 2 and 4. On the other hand, benzoisothiocyanate did not induce subunit 1, but enhanced isoenzyme 2-2. 3-methylcholanthrene increased the relative amounts of isoenzymes 3-3 and 3-4 as compared to isoenzyme 4-4. Inducers for particular isoenzymes are not necessarily substrates, however.

The role of the various isoenzymes of GST in conferring resistance to toxins has been confirmed using expression of recombinant GST isoenzymes in mammalian cell transformants; Puchalski, R.B., et al., Proc Natl Acad Sci USA (1990) 87:2443-2447. This report showed that each of three full-length cloned GST CDNAS--π (acidic), Ya (basic) and Yb₁ (neutral)--conferred drug resistance when expressed in cultured mammalian cells. It was found that GST Ya conferred the greatest increase in resistance to chlorambucil and melphalan; Yb₁ conferred the greatest increase in resistance to cisplatin, and π conferred the greatest increase in resistance to racemic benzopyrene mixtures and to doxorubicin. None of these conferred resistance to vinblastine.

The association of specific GST isoenzymes with tumors has also been studied. Wiencke, J.K., et al., Cancer Res (1990) 50:1585-1590, further describes the art-recognized association of genetic deficiency in the µ isoenzyme of GST with increased lung cancer risk. This deficiency was found to be associated with sensitivity to trans-stilbene oxide-induced cytogenetic damage. The Castro et al. paper, referenced above, also clearly shows the differences in GST complement, depending on the particular tumor cell line being considered.

The GST π isotype has also been associated with tumors, including cancers of the colon, stomach, pancreas, uterine cervix, renal cortex, adenocarcinoma of the breast and lung, nodular small cell lymphoma, mesothelioma, small cell and nonsmall cell lung carcinoma and EJB bladder carcinoma as well as in CLL (Ketterer, B. et al. in "Glutathione Conjugation: Mechanisms and Biological Significance"; Sies, H., et al. eds. (1988) Academic Press, London, pgs. 74-137; Schisselbauer, J.C., et al., Cancer Res (1990) 50:3562-3568). It is not considered that the π class of GST is a marker for particular carcinomas or tumors; however antibodies to π GST said to be useful for detecting cancer are disclosed in PCT application WO 90/12088.

The importance of GST in detoxification mechanisms and drug resistance and the availability of a substantial family of isoenzymes which is unevenly distributed as to its members with respect to normal and tumor tissue, combined with differences between the family members in substrate specificity and inhibitor sensitivity, makes this family an important target for use in designing therapies for conditions associated with malignancy or other unwanted tissue characterized by a discernible GST complement.

### Disclosure of the Invention

The invention is directed to therapeutic modulators and methods of treatment using them which affect the relative efficacy of drugs on normal and malignant or other unwanted tissue. The invention also provides methods to determine GST isoenzyme patterns in normal and malignant tissues, which permits the choice of the appropriate therapeutic modulator.

Thus, in one aspect, the invention is directed to methods to determine the GST isoenzyme complement of separated or unseparated tissue samples, which method comprises preparing Survey of Characteristics (SC) profiles of the separated or unseparated samples and comparing them with the SC profiles of standards containing purified GST isoenzymes, or to mixtures of GST isoenzymes, including those derived from reference tissue samples. The SC profiles are comprised of a number of information channels that represent values of characteristic parameters for a standard or unknown sample. The reagents for the SC profiles may be antibodies which differentially cross-react with the isoenzymes or advantage may be taken of other differentiating properties of these dimers, such as electrophoretic mobility in the presence of various substrates and inhibitors. A particularly useful type of panel comprises a panel of systematically diverse reagents which mimic the binding of the paratope region of antibodies for the various isoenzymes. A detailed description of the nature of paralog panels is set forth in PCT application WO 91/06356.

Whatever the choice of specifically reacting materials or characteristics to provide the reference and test SC profiles, the combination must be such that the comparison results in a characterization of the substrate specificity characteristics, inhibition characteristics, etc., which affect the design of therapeutic regimes and modulators and takes account of the polymorphism and mutability of the GST isoenzyme complement in the population.

In addition, two specific improvements in determining an SC profile are a part of the invention-- the use of a diverse complement of paralogs as affinity ligands in a panel of chromatographic supports and the use of modified glutathione substrates as affinity ligands.

Another aspect is directed to methods of treatment of malignant or other unwanted tissue which tissue has been determined to have a GST complement different from that of normal tissue, and wherein the relevant characteristics of the GST complement have been ascertained. A number of general strategies can be employed in accordance with the methods of the invention. In each of these strategies, the choice of the substances utilized with a particular subject will depend on the outcome of the SC profile determination of the unwanted tissue to be treated. The determination of the SC profile according to the method of the invention thus permits accurate prediction of the outcomes using known drugs and regimens as well as the design of new drugs and new regimens which will be successful in these approaches.

In a first strategy, the cytotoxic drug is a toxin which is inactivated by the GST complement present in normal tissue, and also by a different GST complement which characterizes the unwanted tissue. A therapeutic modulator is supplied along with the toxin. The therapeutic modulator is an inhibitor for the GST specific to the unwanted tissue or is an inducer or activator for the GST in normal tissue. Either approach enhances toxicity in unwanted as opposed to normal cells.

In a second strategy, the cytotoxic agent is generated by a prodrug that is converted by the GST characteristic of the unwanted tissue to a toxic material; this is administered along with a therapeutic modulator which is an inducer or activator for the GST complement characteristic of the unwanted tissue or an inhibitor for normal tissue GST complement.

In a third strategy, a toxin is administered which is unaffected by unwanted-tissue GST but detoxified by GST found in normal cells. The toxin, however, is selected in accordance with the GST profiles.

In a fourth strategy, the redox status of the unwanted tissue, especially a hypoxic tumor, is altered in favor of a less hypoxic state by administration of a substrate for the tumor GST thereby consuming GSH. The substrate is chosen as one which is not effective with the preponderant GSTs in normal tissue. A therapeutic modulator is used in conjunction with this treatment.

All of the foregoing strategies can be practiced in concert with additional therapeutic regimes designed to ameliorate the malignant or other unwanted condition.

In additional aspects, the invention is directed to pharmaceutical compositions useful in conducting the strategies set forth above, and to reference SC profiles for GST isoenzymes obtained by the method of the invention.

### Brief Description of the Drawings

Figure 1 is a xerographic copy of a photograph of a silver stained SDS gel used to separate the GST isoenzymes of various tumor cells. This figure appears in Castro et al., Carcinogenesis (1990) 11:1569-1576.

Figure 2 is a graphical display of the principal components of a distribution of specificity properties of various GST isoenzymes resulting in their classification. This Figure appears as Fig. 1 in Mannervik *et al., Proc Natl Acad Sci USA* (1985) **82**:7202-7206, where its legend is:
"Pattern recognition analysis of enzymatic properties of glutathione transferase isoenzymes from rat, mouse and man. The first, P(1), and second, P(2), principal components computed for each isoenzyme by multivariate analysis (35) on the basis of specific activities with 9 substrates and IC₅₀ values for 11 inhibitors are plotted. The symbols were chosen to designate class alpha (□), class mu(Δ) and class pi (ο)"

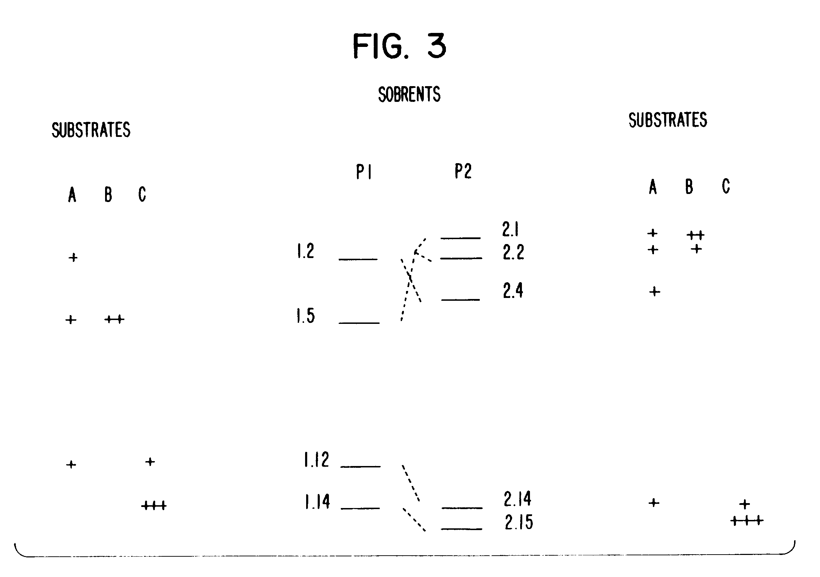

Figure 3 shows a diagrammatic representation of hypothetical results to illustrate the types of profiles that can be obtained. The elution pattern of the GST isoenzymes is matched with a reactivity profile for each, thus combining advantageous features of the prior art methods illustrated in Figures 1 and 2.

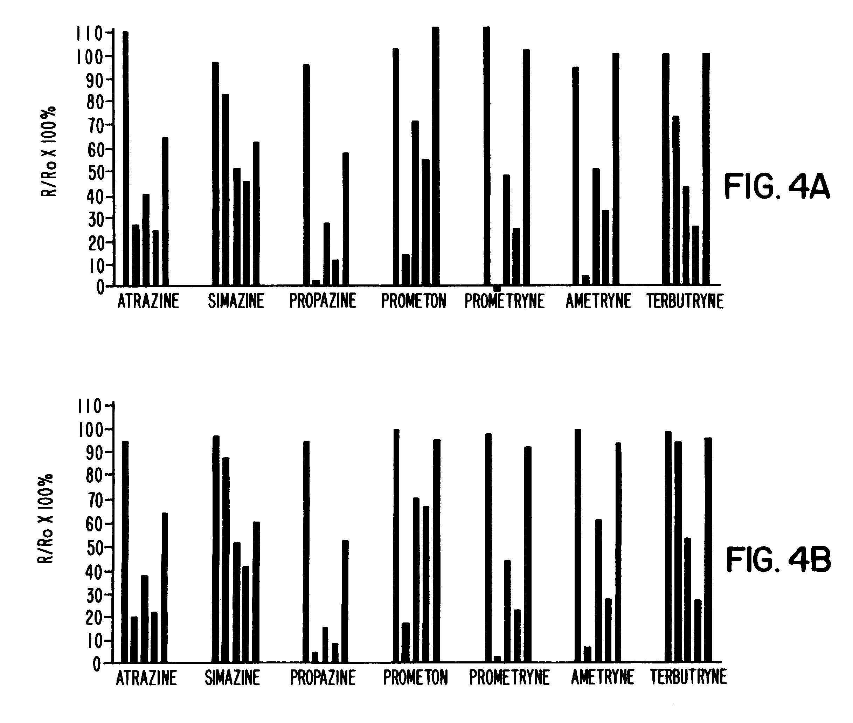

Figures 4A and 4B show typical SC profiles for an illustrative set of analytes.

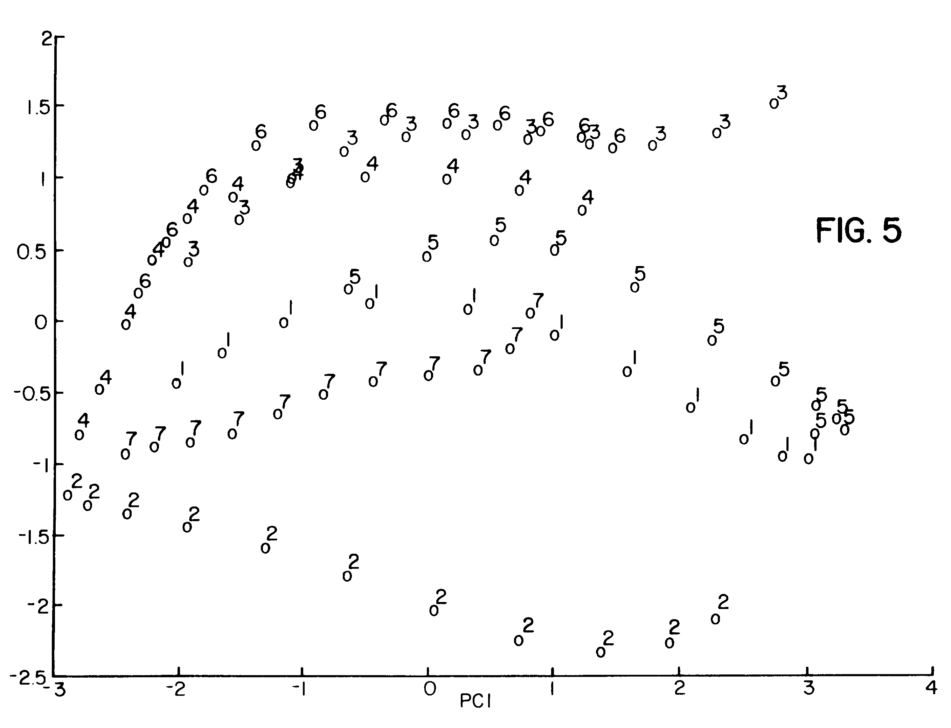

Figure 5 shows a 2-dimensional projection of the plot of SC profiles in 5-dimensions.

### Modes of Carrying Out the Invention

The invention methods take advantage of the differing complements of the glutathione-S-transferase (GST) isoenzymes as they occur in normal (as compared to unwanted) cells or tissues. By "GST complement" is meant the pattern of levels of GST isoenzymes that is present in such cells or tissues or which is genetically programmed in such a manner that induction or repression of expression levels of such GSTs is manipulable. As explained in the background section above, GSTs are homo- or heterodimers formed from subunits, of which at least seven are well known. In addition, although these isoenzymes have been classified broadly, individual members within each class may differ from individual to individual due to genetic variation. The properties of the various isoenzymes differ with respect to a series of measurable parameters including substrate specificity, susceptibility to inhibition, binding to specific reagents, and inducibility of expression.

Perhaps the most easily understood approach to determining the GST isoenzyme complement of an unknown tissue sample presumes a reference set of all GST isoenzymes which have reactivity profiles that have been or can be determined. Thus, assuming a high enough resolution separation, for example, using any separation technique, analogous, for example, to the high resolution chromatographic focusing described by Vos above, an elution pattern is referenced to a series of known enzymes. Other methods for separating and characterizing the known isoenzymes could include the use of antibodies that have been prepared to specific isoenzymes, such as those established for several GST human isotypes and used to assess GST content of these isotypes in candidate tissues (Howie, A.F., et al., Carcinogenesis (1990) 11:451-458; Beckett, G.J. Clinica Chimica Acta (1984) 141:267-273). Gel electrophoresis separations can also be used. The location of the GST bands following electrophoresis under nondenaturing conditions can be determined, for examples, by the method of Ricci, G., et al., Anal Biochem (1984) 143:226-230. The location of various isoenzymes resulting from chromatographic separations can be detected using substrates common to all isoenzymes, such as l-chloro-2,4-dinitrobenzene (CDNB). Indeed, the distribution of activity as assayed by CDNB in various tissues has been conducted by Pickett, C.B., and Lu, A.Y.H., Ann Rev Biochem (1989) 58:743-764.

The use of these direct separation methods to obtain a pattern of GST isoenzyme distributions in cells and tissues of interest can be used to obtain a GST complement for such cells and tissues that may be useful in the design of therapy, provided that each of these isoenzymes has a reactivity profile which has been determined previously following separation techniques permitting isolation of the individual isoenzyme with retention of activity. Such a reactivity profile would take account of the substances which are effective substrates, substances which are effective inhibitors, and substrates which are effective inducers or activators of GST activity. Once the GST isoenzyme is identified and quantitated by virtue of its position in the elution pattern or electrophoretic gel, for example, reference is made to the reactivity profile of the known and previously isolated isoenzyme in order to predict or design treatment protocols such as those described herein.

This method, while readily comprehensible, is not practical due to the large number of GST isoenzymes that are potential candidates for inclusion in the complement and due to the mutability of the repertoire of GST isoenzymes per se. Thus, a polymorphism in the population of available GST isoenzymes is likely to result in a protein with unaltered mobility, for example, in the elution pattern, but with altered substrate specificity or inhibition pattern, or vice versa. In either case, the results of the matching of the position in the elution pattern to the set of reference characteristics would give misleading results.

A somewhat improved result can be obtained by utilizing multiple separation techniques, it being less likely that mobility would be unaffected in multiple separation systems as compared to one. Such a system is illustrated in Figure 3 which indicates that on sorbent P1 four isoenzyme peaks are obtained in the elution pattern; five are obtained in P2. The substrate specificity patterns (for substrates A, B and C) indicate that peak 1.5 is substantially the same in substrate profile as peaks 2.1 and 2.2 separated on sorbent P2; the isoenzyme that elutes at position 1.2 in sorbent P1, according to substrate specificity pattern, elutes at position 2.4 in sorbent P2. Correlations are also shown between peaks 1.12 and 2.14 and peaks 1.14 and 2.15, again using substrate specificity as a criterion.

One would therefore assume that a tissue sample which provided a peak at 1.2 in sorbent P1 and at 2.4 in sorbent P2 would be highly likely to have a reactivity profile wherein A was the only active substrate, and at a relatively low level.

If the separation technique preserves enzymatic activity, the reactivities of each enzyme against potential drugs can be directly determined. Nondenaturing separations in the art, however, suffer from either a lack of resolution or from hypersensitivity to structural changes, making peak identification too problematic for effective guidance of therapy. Ion exchange chromatography, for example, can be used as a step to purify individual GST isoenzymes, but has poor resolution as an analytical tool. IEF, another technique available in the art, is prone to generation of numerous extraneous peaks due to in vivo or in vitro posttranslational modification, and there is no necessary linkage between such structural changes and functional changes.

Thus, determination of the activity profile of the GST complement in cells or tissues by separating the individual isoenzymes using prior art methods and determining an activity profile for each of them against all possible chemotherapeutic drugs appears impractical. One factor in this impracticality is the small amount of tissue typically available.

DNA-based probes for detecting GST components may contribute to defining SC profiles, but are generally inadequate because an inordinate number of probes would be needed to detect the numerous variant forms found in the human population. For each of them, a separate determination of enzymatic activity would also be needed for correlating with DNA information.

A more efficient approach, and that of the invention, takes advantage of profiles of GST isoenzyme complements which simultaneously measure specific binding activity and reactivity characteristics. These profiles, designated survey of characteristics profiles, or "SC" profiles, permit the determination of a reference set of SC profiles which include information on substrate specificity, induction in response to specific inducers, and the like, as well as additional binding or electrophoretic mobility characteristics. By applying computational methods to comparison of these profiles, the requisite information for the design of therapeutic modulators and accompanying protocols and for prediction of success or failure of proposed protocols can be obtained for significantly larger numbers of specimens than by prior art methods, as needed to provide an adequate guide for therapy.

In this approach to determining the GST complement of unknown samples of cells and tissues, advantage is taken of pattern recognition techniques. In connection with this approach, what is here termed an SC profile is determined with respect to a panel of reagents which react specifically and differentially with the various GST isoenzymes. This is analogous to the determination of CRIM profiles obtained by cross-reaction immunoassay, and refers to any pattern of reactivity of a candidate GST isoenzyme or mixture of isoenzymes with a panel of reagents. Thus, the SC profile may be determined with respect to turnover rates for various substrates; effective levels of inhibitor concentration for various inhibitors; levels of inducers required to induce the expression of the gene for the GST isoenzyme in the context of a particular host cell; mobility in electrophoresis in the presence or absence of inhibitors or substrates; elution times from paralog or other affinity columns or, indeed, the classical pattern of binding with a panel of antibodies. The SC profiles obtained for individual GST isoenzymes or mixtures of isoenzymes at various concentration levels can be manipulated in various ways, especially as described by the herein invention, to provide a reference set against which SC profiles of unknown samples can be compared.

In general, the SC profile will provide values for each of a panel of "information channels" wherein each information channel describes a characteristic of the GST complement or standard, such as the binding affinity for an antibody, a substrate affinity, an elution time or the like. At least some of the information channels should relate to values that vary with concentration of the GST.

In order to be useful in the design of embodiments of the strategies to impair or destroy unwanted tissue as provided by the invention, the SC profiles must provide information related to GST activity so that differences between normal and unwanted tissues can be determined. Thus, the SC profiles of the unwanted tissue must be readily comparable to the reference standards which, in turn, must at least in part be based on reactivity patterns that will aid in the design of therapeutic modulators and the selection of drugs or prodrugs. At least a portion of the reference profiles must be grounded in substrate turnover rate data, inhibition data, or data relating to induction of isoenzyme production level, or any other reactivity which will permit manipulation of the GST isoenzyme in situ. Indeed, the combination of paralog chromatographic separation which permits activity to be retained along with preparation of SC profiles with regard to reactivity-affecting reagents for these standards is an important element of the herein invention.

The SC profiles of the reference standards and of the unknown samples are determined with respect to panels of "specifically reactive reagents." These reagents may include a variety of substances, including paralogs, substrates, inhibitors, inducers, antibodies, as well as "reagents" which are actually techniques such as gel electrophoresis or affinity chromatography where the extent of reactivity is determined as electrophoretic mobility or elution time. Thus, "specifically reactive reagents" is not limited to those reagents which effect a chemical reaction, but includes any reagent or technique that permits a characteristic parameter to be obtained for the sample with respect to that reagent.

By "unwanted cells or tissues" is meant any living material which is deleterious to the organism. Perhaps the most prominent example of such unwanted tissue is malignant or even benign tumor tissue. However, under specified conditions it is also desirable to rid the subject of superficial infections such as warts or acne, to destroy hair follicles in the case of, for example, unwanted facial hair, to attack infectious agents such as bacteria and viruses, or to destroy varicose or spider veins. GST in parasites, plants and fungi are useful in the development of fungicides, pesticides, and the like.

Other examples of "unwanted" cells or tissue include those which are putatively normal but which have been modified by the presence of factors attributable to disease conditions so that the putatively normal cells are abnormally affected by these factors. Such "unwanted" cells include those responsible for the production of melanin in hair, where alteration in the GST complement may result in failure to produce melanin and the dopaminergic neurons adversely affected by metabolites generated by Parkinson's disease.

While the side effects of many of the toxins and drugs conventionally employed for antitumor therapy make them undesirable for less deleterious, though nevertheless unwanted, conditions, milder forms of these drugs may be appropriate for the primary treatment in these cases. The enhancement of the therapeutic index (effectiveness of the drug in destroying or impairing unwanted tissues or cells as compared to its effectiveness in destroying normal tissue or cells) which is effected by taking account of the GST complement, including the administration of an appropriate therapeutic modulator, permits the use of milder drugs or lower dosages. Thus, even drugs which according to their current practice are burdened with unacceptable side effects may be usable in conjunction with the therapeutic modulators of the invention, and drugs which are insufficiently potent under present protocols can be employed when their toxicity to unwanted cells or tissues is enhanced by the modulators of the invention.

### Determination of Reference SC Profiles

While some reference standards, as described below, can be prepared directly from normal tissue of a particular subject, it is also useful to provide a databank of SC profiles for a variety of previously isolated GST isoenzymes with characteristic reactivity patterns. By matching these reactivity patterns with those from biopsy samples of the unwanted tissue, the appropriate design for therapeutic modulators and choice of prodrugs or toxins can be made.

U.S. Patent 4,963,263 and PCT application WO 91/06356 describe panels of paralog affinity reagents that are useful in chromatographic separations of closely related substances. Paralog panels can be conveniently used in the preparation of affinity supports for the separation of various GST isoenzymes in purified form while, in each case, retaining the activity of the native isoenzyme. Unlike the reverse-phase HPLC or Western blot methods of the prior art, the separated isoenzymes prepared using paralog chromatography behave in a manner virtually precisely similar to that of the isoenzymes as they occur in nature. For each such purified isoenzyme, then, a SC profile with respect to reactivity of substrate or other activity-affecting reagent can be constructed. A helpful databank of a large number of SC profiles characteristic of these purified isoenzymes can then be retained and stored in mathematically or computationally accessible form for comparison to samples obtained from the unwanted tissue.

In addition, the purified GST isoenzymes can be used to design novel therapeutic modulators by employing them to screen various candidate substances, as described generally in U.S. Patent No. 5,300,425 filed 6 December 1989.

Suitable paralogs can be based on variations such as those, for example, in the various GSH analogs prepared by Adang, A.E.P., et al., Biochem J (1990) 269:47-54, which interact with the various isoenzymes at different concentrations. These analogs are modified forms of GSH in which at least one of the glycine, cysteine, or gamma-glutamine residues is replaced by an alternate amino acid residue. Conjugation of known isoenzyme specific substrates to these glutathione variants or conjugation of these directly to sorbent further increases the systematic diversification of binding properties. In applying these diverse paralogs, profiles for standards and unknown samples can be obtained and compared using banks of the paralogs in appropriate configurations, such as in a panel of affinity columns in which these substances behave as affinity ligands.

### Determination of GST Complement

The invention methods require knowledge of the GST complement of the unwanted cells or tissue in comparison to the normal cells or tissue of the subject, or in comparison to the standard reference panel above. In general, two different approaches can be made to determine these complements. First, using general techniques presently practiced in the art, the individual isoenzymes contained in the sample can be separated using paralog-based affinity supports and tested individually for their patterns of activities. If the paralogs are properly chosen, the individual isoenzymes from the tissue can be obtained and then independently assessed for their substrate specificity, inhibitor specificity and for identifying substances which induce the activity or production of the isoenzyme.

In a second, less laborious, approach, pattern recognition techniques are employed to obtain an instant readout for samples of either or both unwanted and normal tissue for an individual subject by matching these patterns against a reference set prepared as above. In this approach, less volume of sample is required and no separation is necessary. This method is also useful when applied to tissue slices using histochemical staining for GST activity.

Turning, first, to the paralog-based separation approach, a modification of the separation method used by Vos, R.M.E., et al., Biochem Pharmacol (1988) 37:1077-1082, discussed in the background section above, can be used. In this method, the cytosol fraction frdm a complete rat liver was subjected to an affinity column of S-hexyl GSH Sepharose™ used as an affinity reagent for GSTs as a group. The eluted GST mixture was concentrated and separated by chromatofocusing on a mono-PHR 5/20 column (Pharmacia FPLC system). The individual isoenzymes were collected in separate fractions and analyzed. Fractions were identified by their position in the elution profile, their subunit molecular weight, and specific activities toward 1-chloro-2,4-dinitrobenzene, which is a substrate for most known GSTs.

As modified by using the paralog technology referenced above, milder conditions can be employed, and more active forms of the GST isoenzymes can be recovered. These are then tested for substrate specificity, etc.

As described above, one might consider the possibility of simply using arbitrary separation technology such as that of Vos that provides an elution pattern characteristic of the various GST isoenzymes, and matching the elution pattern for cells or tissue of unknown GST complement with the preset elution pattern to determine the nature of the GST complement in the unknown. One problem with this approach, however, lies in the genetic mutability of GST, so that it is difficult to make reliable matches that will retain the inferred characteristics and thus be assured to have similar reactivity patterns. The genetic mutability of the isoenzymes as well as their sensitivity to posttranslational modifications is very likely, in any particular case, to have a profound effect on the substrate specificity, inhibition patterns, and the like, as well as in binding and physical characteristics, such as pI. There is no guarantee that a correlation will exist between reactivity variation and physical property or binding variation; indeed, the probability is that the effects will not be correlated.

In the pattern-matching approach, a more reliable assay is conducted by comparing the profile of reactivity of an unknown sample with a set of reference SC profiles. A predetermined plot of SC profiles obtained from samples of known GST complement is used as a reference with which an SC of the sample to be tested can be compared. Generally, a panel of 2-10, preferably 4-6, different specifically reactive reagents is first used to provide profiles for samples of known compositions. In one embodiment, specific binding assays are used where there is cross-reactivity by the GST isoenzymes across a panel of binding agents, and the profiles are then obtained by measurement of inhibition values for binding of a known binding partner by various GST isoenzymes. The collection of profiles is then treated mathematically by any of a number of techniques to generate a readable comparison with the corresponding SC profile of an unknown sample.

While a convenient parameter involves reactivity with a binding reagent, such as an antibody, typically a monoclonal antibody, this is merely an illustration and not a requirement. For example, any substance which is determined to react generally with the group of GST isoenzymes is usable in the method of the invention. -Antibodies, including, for example, single chain antibodies and recombinantly produced antibodies, can be used per se, or as immunologically reactive fragments thereof, as is well understood in the art. The use of, for example, Fab, Fab' or F(ab')₂ fragments is often convenient in specific binding assays. Any reagent interaction which provides suitable cross-reactivity can be used, such as enzyme-substrate or enzyme-inhibitor binding, ligand-receptor binding, or binding to an affinity reagent such as the paralogs described in U.S. patent 4,963,263.

In addition to reactivity through specific binding, any type of reactivity can be measured. For example, for the GST isoenzymes, the effect on activity of each of a panel of inhibitors could be measured, or the level of activity with regard to a series of substrates could be measured. It is not necessary that the same type of reactivity or other characteristic parameter be used to determine a value for each information channel of which the profile is comprised. Thus, the profile may constitute a combination of binding with an antibody, binding with an inhibitor, reactivity with another enzyme, reactivity with a substrate, or any other chemical activity or physical characteristic which has a variable value for the various GST isoenzymes to be determined.

Similarly, the choice of specific assay format which detects the reactivity of the reagents in the panel or value of characteristic parameter in the panel that provides the series of information channels for the profiles of the GST isoenzyme group members is optional. Binding or other reactivity of the sample to be tested and the production of a standard set of data points for various GST isoenzyme compositions can be determined in either a direct or competitive format. For example, a binding agent can be labeled and the binding to antigen detected by precipitating the resultant complex and determining the amount of label in the precipitate. Alternatively, and more conveniently, a binding reagent is coupled to a solid support and the GST isoenzyme allowed to compete with a reagent of known binding capacity. Additional methods for conducting assays designed to detect and measure binding of a specific binding reagent to the analytes in a particular group are disclosed in PCT application US88/03554. Methods for design of specific binding reagents are also found in U.S. Patent No. 4,963,263 and in PCT US89/01194 and PCT US90/06333. Alternative protocols will be immediately apparent to practitioners of immunoassays or other specific binding assays.

Similarly, methods to determine parameters which measure chemical reactivity or physical characteristics are also readily devised. For example, the relative mobilities of the isoenzymes in a chromatographic support constitutes a differential cross-reactivity to provide information for one channel. Electrophoretic mobilities, pI values, reactivity with particular substrates or inhibition by particular inhibitors also constitute parameters which provide information for the profile.

However the value of the characteristic parameter for each information channel is determined, a profile for a panel of "cross-reactive" characteristic parameters is determined for a series of known GST isoenzyme compositions. The simplest such compositions, and those typically used to obtain the standard pattern, are samples containing only a single isoenzyme at a range of concentrations. Calculation of plot positions for mixtures is also possible.

The profiles determined for the isoenzyme standards, and unknown compositions are somewhat analogous to cross-reaction immunoassay (CRIM) profiles and the method of the invention is analogous to comparing the CRIM profile of a sample to be tested with a predetermined plot of CRIM profiles obtained from samples of known analyte composition. In the invention method, the concept of a CRIM profile is broadened to include a series of channels of values of characteristic parameters such as specific reactivities which include not only reactivities with antibodies or binding agents, but also reactivity in a variety of contexts and any informational physical or chemical characteristic which has a differential value across the GST complement. For convenience, because the number of characteristics which could be surveyed is large, this profile is designated herein a survey of characteristics profile or "SC profile."

Thus, by "SC profile" is meant a pattern of values of characteristic parameters across a panel of information channels provided by reagents or a set of physical or chemical characteristics with respect to a single fixed GST isoenzyme composition. In a typical SC profile useful in the invention, the characteristics of the GST isoenzyme with respect to 2-10 preferably 4-6 different information channels are compiled. For example, the reactivity of a sample with a panel of 2-10, preferably 4-6, different reagents can be determined. As will be apparent from the example below, each composition will have a characteristic SC profile across the panel of information channels comprised of reactivity with reagents and/or other characteristics. Larger numbers of members of the panel provide greater refinement of the assay; smaller numbers of members of the panel are more convenient. The choice of the number of members of the panel is arbitrary, depending on the level of fine tuning desired in the assay; the mathematical techniques disclosed herein permit selection of the most meaningful panel members and can be used to reduce the number of information channels needed in the profile.

In obtaining the profiles at various concentrations, the simplest conceptual approach provides this series of profiles by direct measurement of the inhibition values at various known isoenzyme concentrations with respect to binding reagents or enzymatic activity. However, additional profiles can be interpolated using the curves obtained by plotting % inhibition vs. isoenzyme concentration.

The profiles obtained for the individual standard compositions of GST isoenzymes to be compared to unknown samples are then subjected to computational techniques which permit the comparison of the standard profiles with those of unknowns, a process not readily performed by hand.

In the simplest form of application of these pattern recognition techniques, each SC profile is plotted as a point in n dimensions, wherein n is the number of binding agents in each panel.

For example, one might use a panel containing six different binding agents which are monoclonal antibodies. These antibodies are assumed to be cross-reactive with, for example, ten isoenzymes, Aᵢ, wherein A represents the isoenzyme and i is an integer of 1-10. One of these isoenzymes, A₁, for example, might be chosen as a labeled competitor to determine profiles of competition for binding at various concentrations of itself and of the remaining isoenzymes. Using a set concentration of labeled A₁, the percent inhibition, for example, is determined with respect to binding each antibody in the panel at various concentrations of A₁-A₁₀.

For each Aᵢ, at one concentration, then, there are six data points which are percentages of inhibition with respect to labeled A₁ for binding to the various antibodies in the panel. These percentages are then treated mathematically as defining the location of a single point in six-dimensional space where the first dimension describes the percent inhibition with respect to the first antibody, the second dimension describes the percent inhibition with respect to the second antibody, and so forth. Points are thus determined representing the various concentrations of A₁, A₂, A₃ . . . A₁₀.

As a six-dimensional plot is not readily visualized, known mathematical techniques, such as those described by D.L. Massart, et al., "Chemometrics: A Textbook" (1988) Elsevier, (N.Y.), can be used to project the six-dimensional array onto a two-dimensional surface or other surface of lower dimensionality. This series of points in two-dimensional space can then be used to visualize comparisons of the profile obtained using an unknown sample with the profiles of standards to identify the isoenzyme composition of the sample. Of course, as the six-dimensional space can be handled mathematically, there is no requirement for the projection in order to match the data point generated by the samples with those in the set of reference points.

Choosing a projection does, however, provide the additional benefit of rank ordering the antibodies with respect to their utility for SC. The more nearly perpendicular the projection plane is to an axis in the six dimensional space, the greater is the loss of that component's information content in the projection. Since factor analysis generates an optimal plane for preserving the scattering aspect of that information, the relative importance of the antibodies in this regard is readily determined. This knowledge may be usefully applied, for example in reducing the number of antibodies in the panel.

In the example described below, it was found that reliable estimates could be obtained for compositions of an unknown sample in about 85% of the cases using this rather simple mathematical approach. Improved results were obtained by applying methods to distinguish between data points which are of significance from those which are relatively meaningless.

In effect, weighting factors for the various members comprising the profile can be introduced to account for the fact that those concentrations which represent inhibition of binding on the linear part of the standard curve are more informative than those in the asymptotic parts of the curve at very low or high inhibition. These factors are applied when the data are treated by "variance analysis." In this technique, in general outline for this example, the value represented by the dimension corresponding to each of the six monoclonal antibodies in the panel is compared separately with the corresponding value for the corresponding antibody in the unknown sample, rather than as a totality of the n-dimensional result for all six antibodies.

For example, the observed inhibition value for antibody #1 implies a corresponding concentration for A₁, and a different corresponding concentration for A₂, etc. Antibody #2 similarly generates a family of predictions, and so forth. For the correct choice of isoenzyme, the individual predictions of the six antibodies will agree more closely than for an incorrect choice of isoenzyme. This approach to profile identification is a form of variance analysis, normally used to compare independent estimates made in different laboratories or methods of determination. Calculation of variances can be easily modified to weight the predictions by a factor representing the reliability of the data, as judged by the variance in the data set used to construct the standard curve. This procedure is referred to herein as "weighting the results."

This mathematical technique, as described by Mandel, "The Statistical Analysis of Experimental Data" (1984) Dover, provided sufficient weighting of the significant binding data to provide a clear result in 95% of the cases.

Finally, neural net systems can also be used, wherein adjustment factors arise implicitly in the process of training the net's input/output characteristics using standards. The systems are outlined, for example, in Hartz, J.A., et al., Introduction to the Theory of Neural Computation (Addison Wesley, Santa Fe Institute Series on Complex Systems, 1991); Parallel Distributed Processing, 2 vols. (D.E. Rumelhart and J.L. McClelland, eds., MIT Press, 1986); or DARPA Neural Network Study (Armed Forces Communication and Electronics Association Int'l Press, 1988).

An illustration of the application of these techniques for determination of SC profiles and evaluation of the profiles in unknown samples is as follows. This example uses triazines, but analogous techniques may be used for GST complement determination.

A series of standard profiles for each triazine analog over the 10-1000 ppb range was calculated from the inhibition curves obtained for each of the triazines used. The result of this theoretical calculation for 50 ppb is shown in Figure 4A. As seen, the percentage inhibition patterns across the panels is distinctive for each individual triazine.

The profiles were also determined experimentally at 50 ppb, as shown in Figure 4B. The calculated and experimental patterns are highly similar.

Matching experimental profiles to calculated reference profiles allows identification of unknown samples. A catalog of calculated profiles thus provides a reference for unknown sample determination.

Profiles such as those shown in Figure 4A were determined for a series of concentrations of seven triazines in the 10-1000 ppb range. The percent inhibition values for the five antibodies for each triazine at each concentration provides a coordinate in 5-dimensional space. Thus, each analog at each concentration gives a single point representing the profile in 5-dimensional space.

The resultant pattern shows a unique series of points represented by individual analytes at various concentrations with respect to the antibody panel.

In order to visualize these data better, the 5-dimensional plot is projected into a 2-dimensional array, as shown in Figure 5.

The orientation of the two-dimensional plane used for this projection is that which best preserves the scatter of the data, i.e., which minimizes points lying on top of each other in the projection when they are well separated. This orientation is defined mathematically as the plane specified by the principal components of the data. Projecting points onto the plane defined by the principal components preserves the clustering and characteristics of the original data as described by Massart, D.L., et al., "Chemometrics: A Textbook" (1988) Elsevier, New York.

As shown in Figure 5, all points labeled "1" represent various concentrations of atrazine, all points labeled "2" represent varying concentrations of simazine, and so forth.

By determining the point location resulting from obtaining the profile of a sample in the same manner as described for known compositions in this example, the analyte composition of the sample can be obtained.

For use in determining the GST complements needed to practice the therapeutic methods of the invention, the analogous SC profiles can be determined using either isolated GST isoenzymes or mixtures thereof which contain known compositions or both. The specifically reactive reagents must, in this case, include at least some members which give guidance as to the types of drugs, prodrugs, or therapeutic modulators that would be useful in treating the unwanted tissue. The panel members of the specifically reactive reagents can include, for example, a series of known substrates and in this case, the profile might be set forth as a pattern of turnover rates with respect to these substrates. Suitable substrate candidates include, for example, ethacrynic acid, bromosulfophthalein, cumene hydroperoxide, BCNU, chlorambucil, trans-stilbene oxide and the like.

Also available for use as specifically reacting reagents which are members of the panel to provide a SC profile are inhibitors which interact with the GST isoenzyme at various levels. These inhibitors include, for example, piriprost, Cibacron Blue, and hematin. Antibodies which are specifically immunoreactive with the various isoenzymes can be used, as well as paralog-type affinity reagents. However, at least some members of the panel must be descriptive of the enzymatic activity of the GST in order to provide a basis for therapeutic strategy design.

An additional technique for obtaining SC profiles is analogous to that described by Takeo, K., et al., J Immunol (1978) 121:2305-2310. In this approach, differential electrophoresis in the presence of various binding agents for the individual proteins permits measurement of a mobility value. In the specific application described by Takeo, measurements of dextranspecific myeloma proteins in polyacrylamide gel electrophoresis were made, showing retardation when the dextrans were added to the separating gel, which retardation could be reversed by adding the hapten isomaltose oligosaccharide. In using this approach, a series of mobilities depending on the choice of retarding agent, for example, could be obtained for known compositions. This technique could be adapted to the SC profiles of the invention by utilizing substrates and/or inhibitors as the retarding or mobilizing agents.

In a preferred method for determining the GST complement of biopsies, a series of HPLC columns is constructed using the known GST substrates studied by Mannervik, B., et al., Proc Natl Acad Sci (1985) 82:7202-7206, referenced above. These substrates are conjugated directly to the column supports or are attached to the GSH analog variants described by Adang, A.E.P., et al., Biochem J (1990) 269:47-54, also referenced above. A series of 50-100 different columns resulting from the various possible combinations of substrates with GSH analogs represents a series of candidate sorbents. These sorbents are tested to select those of maximal diversity in properties by utilizing each for the separation of a mixture of known GST isoenzymes. The four or five columns with the greatest differentiation capacities are then chosen as panel members for determining SC profiles in unknown samples and in standards.

Thus, rather than displaying the separations as elution patterns on each individual sorbent, the data are rearranged so that the capacity for adsorption to each sorbent represents an information channel in the SC profile of the isoenzyme. The reactivity pattern with respect to inducers, activators, substrates, and inhibitors are also determined for each isoenzyme and used as an information channel. The completed profile for each known isoenzyme is then used as a member of a reference set. Additional members of the reference sets are determined by utilizing samples from normal tissues and evaluating the values assigned to the same set of information channels. The corresponding profiles of biopsy samples from unknown, unwanted tissues are then compared against this reference set.

Whatever the choice of panel members for use in determining the profiles, the profiles for known compositions are stored in computationally accessible form for comparison to profiles similarly determined for unknown samples. Thus, kits can be provided for determination of the GST complement of unknown samples which include the test panel members used in determination of the reference profiles along with instructions for SC profile determination of the unknowns. Suitable software to access the reference profiles may also be included.

After the GST complement of the unwanted tissue in question has been determined, the appropriate strategy can be selected for treatment to impair or destroy these cells or tissue. The complement may be evaluated to determine whether or not standard treatment protocols will be successful when applied to the unwanted cells or tissues or may be used for the design of different protocols including the choice of toxin or prodrug and the inclusion or noninclusion of a therapeutic modulator.

### Therapeutic Strategies

Three of the four strategies require the use of a cytotoxic drug or a prodrug therefor. A wide range of such possible toxins is known in the art. Of course, the clearance of the toxin must be subject to control by GST in order to take advantage of the differing complements of this enzyme between unwanted and normal tissue.

A review of various cytotoxic agents useful in the invention is found, for example, in a chapter by Docampo, R., et al., in "Free Radicals in Biology" (1984) VI:243-280. This review summarizes the free radicals generated by such drugs as menadione, menoctone, daunorubicin, mytomicin-C, actinomycin-D, 9-hydroxyellipticine, 5-methylphenazinium methyl sulfate, nifurtimox, benznidazole, and the various radiosensitizers metronidazole, secnidazole, ronidazole, and mizonidazole. Various porphyrins and dyes such as chlorpromazine and primaquine are also described. Other related compounds that have an ortho-dihydroxybenzene moiety and are structurally related to levodopa and dopamine are disclosed by Wick, M.M., in J Invest Dermatol (1989) 72:329S-331S. A wide variety of toxins useful in chemotherapy in general is known in the art.

In addition, various prodrugs for toxins are also known. Examples of prodrugs which are activated by various GST isoenzymes include 1,2-bromoethane and azathioprine.

The primary cytotoxic agents or their prodrugs are administered in conventional protocols and in conventional formulations in the strategies that employ them. Generally, systemic administration is preferred, usually by injection, but if desired, by transmucosal, transdermal or oral administration. Suitable formulations for any of these routes may be found, for example, in Remington's Pharmaceutical Sciences, latest edition, Mack Publishing Co., Easton, PA. In some of these strategies, the cytotoxic agent or prodrug is administered along with the therapeutic modulator which enhances or inhibits the desired GST isoenzyme. Suitable known therapeutic modulators are those known to affect GST activity such as ethacrynic acid, 3-methylcholanthrene, Cibacron blue, hematin and the like, as set forth hereinabove. However, additional therapeutic modulators for the specific GST isoenzymes found to characterize the GST complement of unwanted tissue can be found using screening methods as set forth above.

Thus, the toxin may be chosen so as to be inactivated only by the GST of normal tissue, but to be ignored by the clearing GST of unwanted tissue; a prodrug can be chosen that is activated only by the GST in unwanted tissue, not by that in normal tissue. In addition, the native balance of GST activities is tipped by administration of one or more therapeutic modulators which alter the activities of either or both the normal or unwanted tissue GST isoenzymes. Finally, the therapeutic modulator may affect the activity of an oxidizing agent whose oxidizing power is coupled specifically and preferentially through the GST isoenzyme in hypoxic unwanted tissue.

## Claims

1. A method of selecting a therapeutic modulator that affects GST activity preferentially in target cells or tissue, or selecting a toxin which is relatively unaffected by the GST complement of unwanted cells or tissues, or selecting a prodrug that is converted to a toxin by the GST complement of unwanted cells or tissues, the method comprising evaluating said modulator, toxin or prodrug with respect to the GST complement of said target cells or tissue, said GST complement being determined by a method comprising:
evaluating the value of each information channel in a panel of characteristics related to GST isoenzymes of said cells or tissue by contacting said cells or tissue with each member of a panel comprising n specifically reactive reagents that are reactive with at least one GST isoenzyme wherein n is an integer and is at least 2; and
evaluating the reactivity of each specifically reactive reagent in the panel with the cells or tissue to obtain a survey of characteristics (SC) profile for said cells or tissue; and
comparing the resulting SC profile obtained from said cells or tissue with a reference set of SC profiles for the same panel of characteristics obtained from samples of known GST complements.

2. A method according to claim 1 wherein, in the method of determining the GST complement, at least one of said reagents is a substrate, inhibitor, activator or inducer of a GST isoenzyme, or is an antibody or immunoreactive fragment thereof specific for a GST isoenzyme.

3. A method according to claim 1 or 2 wherein, in the method of determining the GST complement, said comparing includes the steps of converting the profile obtained to a point in n-dimensional space by plotting independently in said n-dimensional space the value of each information channel of the panel for the unknown cells or tissues, wherein n is the number of information channels in the panel;
comparing the position of said point to the positions of reference points in said n-dimensional space determined in an analogous manner for samples of known GST complement; and
identifying the GST complement of the unknown cells from the reference point close st to that obtained from the unknown cells or tissue.

4. A pharmaceutical composition comprising a therapeutic modulator that has been selected by a method according to any of claims 1 to 3.

5. A composition according to claim 4 wherein said therapeutic modulator is an inhibitor or an inducer or activator for the GST complement of predetermined cells or tissue.

6. The use of a toxin effective to destroy or impair the functioning of unwanted cells or tissue and a therapeutic modulator selected by a method according to claim 1 effective to render the toxin selectively more susceptible to detoxification by the GST complement of normal cells or tissue as compared to the GST complement of the unwanted cells or tissue for the manufacture of a medicament for treating or ameliorating a condition characterized by the presence of the unwanted cells or tissue.

7. The use of a prodrug that is converted to a toxin by the GST complement of unwanted tissues or cells to a substantially greater extent than by the GST complement of the normal tissues or cells as selected by a method according to claim 1 for the manufacture of a medicament for treating or ameliorating a condition characterized by the presence of the unwanted cells or tissue.

8. The use of a toxin which is relatively unaffected by the GST complement of unwanted tissue or cells but which is detoxified by the GST complement of normal tissue or cells as selected by a method according to claim 1 for the manufacture of a medicament for a treating or ameliorating a condition characterized by the presence of the unwanted cells or tissue.

9. The use of a GST substrate effective to consume the glutathione of unwanted tissues or cells in a reaction mediated by the GST complement of said unwanted tissues or cells, but which substrate does not consume the GSH content of normal tissue or cells, thereby rendering said unwanted tissue or cells sensitive to electrophiles generated by chemotherapy or radiation, in combination with a therapeutic modulator as selected by a method according to claim 1, for the manufacture of a medicament treating or ameliorating a condition characterized by the presence of the unwanted cells or tissue.

## Patentansprüche

1. Verfahren zur Auswahl eines therapeutischen Modulators, der die GST-Aktivität vorzugsweise in Zielzellen oder -gewebe beeinflußt, oder zur Auswahl eines Toxins, das durch das GST-Komplement unerwünschter Zellen oder Gewebe relativ unbeeinflußt ist, oder zur Auswahl eines Prodrugs, das durch das GST-Komplement unerwünschter Zellen oder Gewebe in ein Toxin umgewandelt wird, wobei das Verfahren die Bewertung des Modulators, Toxins oder Prodrugs im Bezug auf das GST-Komplement der Zielzellen oder -gewebe umfaßt, wobei das GST-Komplement durch ein Verfahren mit folgenden Schritten bestimmt wird:
Bewertung des Wertes jedes Informationskanals in einer Reihe von Eigenschaften bezogen auf GST-Isoenzyme der Zellen oder Gewebe durch Inkontaktbringen der Zellen oder des Gewebes mit jedem Mitglied der Reihe, umfassend n spezifisch reaktive Reagenzien, die mit mindestens einem GST-Isoenzym reagieren, wobei n eine ganze Zahl ist und mindestens 2 beträgt; und
Bewertung der Reaktivität jedes spezifisch reaktiven Reagenzes in der Reihe mit den Zellen oder dem Gewebe zum Erhalt eines Eigenschaften (survey of characteristics, SC)-Profils für die Zellen oder das Gewebe; und
Vergleich des resultierenden SC-Profils, das von den Zellen oder dem Gewebe erhalten wird, mit einem Bezugssatz von SC-Profilen für die gleiche Reihe von Eigenschaften, erhalten von Proben von bekannten GST-Komplementen.

2. Verfahren nach Anspruch 1, wobei im Verfahren zur Bestimmung des GST-Komplements mindestens eines der Reagenzien ein Substrat, Inhibitor, Aktivator oder Induktor eines GST-Isoenzyms ist, oder ein Antikörper oder ein immunreaktives Fragment davon, das spezifisch ist für ein GST-Isoenzym.

3. Verfahren nach Anspruch 1 oder 2, wobei in dem Verfahren zur Bestimmung des GST-Komplements der Vergleich die Schritte umfaßt: Umwandlung des erhaltenen Profils in einen Punkt in einem n-dimensionalen Raum durch unabhängiges Auftragen des Wertes jedes Informationskanals der Reihe der unbekannten Zellen oder Gewebe in dem n-dimensionalen Raum, wobei n die Zahl der Informantionskanäle in der Reihe ist;
Vergleich der Position des Punktes mit den Positionen von Bezugspunkten in dem n-dimensionalen Raum, die in analoger Weise für Proben mit bekanntem GST-Komplement bestimmt wurden; und
Identifizierung des GST-Komplements der unbekannten Zellen durch den Bezugspunkt, der am nächsten zu dem ist, der von den unbekannten Zellen oder Gewebe erhalten wurde.

4. Arzneimittel, umfassend einen therapeutischen Modulator, der nach einem Verfahren gemäß einem der Ansprüche 1 bis 3 ausgewählt wurde.

5. Mittel nach Anspruch 4, wobei der therapeutische Modulator ein Inhibitor oder ein Induktor oder ein Aktivator für das GST-Komplement von vorbestimmten Zellen oder Gewebe ist.

6. Verwendung eines Toxins, das wirksam ist bei der Zerstörung oder Schädigung der Funktion von unerwünschten Zellen oder Gewebe, und eines therapeutischen Modulators, ausgewählt nach einem Verfahren gemäß Anspruch 1, der wirksam ist, um das Toxin selektiv mehr empfänglich für Entgiftung durch das GST-Komplement von normalen Zellen oder Gewebe zu machen, verglichen mit dem GST-Komplement der unerwünschten Zellen oder Gewebe, zur Herstellung eines Medikaments zur Behandlung oder Linderung eines Zustandes, der durch die Gegenwart der unerwünschten Zellen oder Gewebe gekennzeichnet ist.

7. Verwendung eines Prodrugs, das durch das GST-Komplement unerwünschter Gewebe oder Zellen in einem wesentlich größeren Ausmaß als durch das GST-Komplement von normalen Gewebe oder Zellen in ein Toxin umgewandelt wird, ausgewählt nach einem Verfahren gemäß Anspruch 1, zur Herstellung eines Medikaments zur Behandlung oder Linderung eines Zustandes, der durch die Gegenwart der unerwünschten Zellen oder Gewebe gekennzeichnet ist.

8. Verwendung eines Toxins, das durch das GST-Komplement von unerwünschtem Gewebe oder Zellen relativ unbeeinflußt ist, das aber durch das GST-Komplement normaler Gewebe oder Zellen entgiftet wird, ausgewählt nach einem Verfahren gemäß Anspruch 1, zur Herstellung eines Medikaments zur Behandlung oder Linderung eines Zustandes, der durch die Gegenwart der unerwünschten Zellen oder Gewebe gekennzeichnet ist.

9. Verwendung eines GST-Substrats, das wirksam ist im Verbrauch des Glutathions unerwünschter Gewebe oder Zellen in einer Reaktion, die durch das GST-Komplement der unerwünschten Gewebe oder Zellen vermittelt wird, wobei aber das Substrat den GSH-Gehalt von normalem Gewebe oder Zellen nicht verbraucht, so daß das unerwünschte Gewebe oder Zellen für Elektrophile empfindlich gemacht wird, die durch Chemotherapie oder Bestrahlung erzeugt werden, in Kombination mit einem therapeutischen Modulator, ausgewählt nach einem Verfahren gemäß Anspruch 1, zur Herstellung eines Medikaments zur Behandlung oder Linderung eines Zustandes, der durch die Gegenwart der unerwünschten Zellen oder Gewebe gekennzeichnet ist.

## Revendications

1. Une méthode de sélection d'un modulateur thérapeutique qui affecte préférentiellement l'activité de la GST dans des cellules ou un tissu cibles, ou de sélection d'une toxine qui est relativement non affectée par le complément de la GST de cellules ou tissus non désirés, ou de sélection d'un promédicament qui est converti en une toxine par le complément de la GST de cellules ou tissus non désirés, la méthode comprenant l'évaluation dudit modulateur, toxine ou promédicament par rapport au complément de la GST desdites cellules ou dudit tissu cibles, ledit complément de la GST étant déterminé par une méthode comprenant :
l'évaluation de la valeur de chaque canal d'information dans un ensemble de caractéristiques concernant les isoenzymes GST desdites cellules ou dudit tissu par mise en contact desdites cellules ou tissu avec chaque membre d'un ensemble comprenant n réactifs réagissant spécifiquement qui réagissent avec au moins une isoenzyme GST dans laquelle n est un nombre entier et est au moins 2 ; et
l'évaluation de la réactivité de chaque réactif réagissant spécifiquement dans l'ensemble avec les cellules ou le tissu pour obtenir une vue globale du profil de caractéristiques (SC) desdites cellules ou dudit tissu ; et
la comparaison du profil SC résultant obtenu à partir desdites cellules ou dudit tissu avec un ensemble de référence de profils SC pour le même ensemble de caractéristiques obtenu à partir d'échantillons de compléments de la GST connus.

2. Une méthode selon la revendication 1 dans laquelle, dans la méthode de détermination du complément de la GST, au moins un desdits réactifs est un substrat, un inhibiteur, un activateur ou un inducteur d'une isoenzyme GST, ou est un anticorps ou un fragment immuroréactif de celui-ci spécifique d'une isoenzyme GST.

3. Une méthode selon la revendication 1 ou 2 dans laquelle, dans la méthode de détermination du complément de la GST, ladite comparaison comprend les étapes comprenant la conversion du profil obtenu en un point dans un espace à n dimensions en mettant indépendamment en graphique, dans ledit espace à n dimensions, la valeur de chaque canal d'information de l'ensemble pour les cellules ou tissus inconnus, où n est le nombre de canaux d'information dans l'ensemble ;
la comparaison de la position dudit point aux positions des points de référence dans ledit ensemble à n dimensions déterminé d'une manière analogue pour les échantillons de complément de la GST connu ; et
l'identification du complément de la GST des cellules inconnues par rapport au point de référence le plus proche de celui obtenu à partir des cellules ou du tissu inconnus.

4. Une composition pharmaceutique comprenant un modulateur thérapeutique qui a été sélectionné par une méthode selon l'une quelconque des revendications 1 à 3.

5. Une composition selon la revendication 4 dans laquelle ledit modulateur thérapeutique est un inhibiteur ou un inducteur ou un activateur du complément de la GST de cellules ou d'un tissu prédéterminés.

6. L'utilisation d'une toxine, capable de détruire efficacement ou d'empêcher le fonctionnement de cellules ou d'un tissu non désirés, et d'un modulateur thérapeutique sélectionné par une méthode selon la revendication 1 capable de rendre la toxine sélectivement plus sensible à la détoxification par le complément de la GST de cellules ou d'un tissu normaux par comparaison au complément de la GST de cellules ou d'un tissu non désirés, pour la préparation d'un médicament destiné au traitement ou à l'amélioration d'un état caractérisé par la présence de cellules ou d'un tissu non désirés.

7. L'utilisation d'un promédicament qui est converti en une toxine par le complément de la GST de tissus ou cellules non désirés à un degré sensiblement plus élevé que par le complément de la GST de cellules ou tissus normaux tels que sélectionnés par une méthode selon la revendication 1, pour la préparation d'un médicament destiné au traitement ou à l'amélioration d'un état caractérisé par la présence de cellules ou d'un tissu non désirés.

8. L'utilisation d'une toxine qui est relativement non affectée par le complément de la GST d'un tissu ou de cellules non désirés, mais qui est détoxifiée par le complément de la GST d'un tissu ou de cellules normaux tels que sélectionnés par une méthode selon la revendication 1, pour la préparation d'un médicament destiné au traitement ou à l'amélioration d'un état caractérisé par la présence de cellules ou d'un tissu non désirés.

9. L'utilisation d'un substrat de la GST capable de consommer le glutathion de tissus ou cellules non désirés dans une réaction induite par le complément de la GST desdits tissus ou cellules non désirés, mais lequel substrat ne consomme pas le contenu en GSH d'un tissu ou de cellules normaux, rendant ainsi ledit tissu ou lesdites cellules non désirés sensibles aux électrophiles générés par la chimiothérapie ou les radiations, en combinaison avec un modulateur thérapeutique tel que sélectionné par une méthode selon la revendication 1, pour la préparation d'un médicament destiné au traitement ou à l'amélioration d'un état caractérisé par la présence de cellules ou d'un tissu non désirés.
